# EUROPÄISCHE PATENTANMELDUNG

(11) **EP 3 939 530 A1**
(43) Veröffentlichungstag der Anmeldung: **19.01.2022**
(21) Anmeldenummer: 21184548.2
(22) Anmeldetag: 08.07.2021
(51) Int. Cl.: A61B 18/14

(54) **CHIRURGISCHES HANDGERÄT, ISOLIEREINSATZ FÜR EIN CHIRURGISCHES HANDGERÄT UND VERFAHREN ZUR HANDHABUNG EINES CHIRURGISCHEN HANDGERÄTS**

(30) Priorität: 17.07.2020 DE 102020118965
(71) Anmelder: Olympus Winter & Ibe GmbH, 22045 Hamburg (DE)
(72) Erfinder: Brockmann, Christian, 21279 Hollenstedt (DE)
(74) Vertreter: Hoener, Matthias

(57) **Zusammenfassung**

Chirurgische Handgeräte werden in der Urologie bei elektrochirurgischen Arbeiten verwendet. Für diese Anwendung wird eine Elektrode mit einem hochfrequenten elektrischen Strom beaufschlagt. Dabei gilt es zu vermeiden, dass die Elektrode in elektrischen Kontakt mit dem Handgerät kommt. Bekannte Isoliereinsätze zur elektrischen Isolierung lassen sich nur schwer mit dem Handgerät verbinden. Außerdem ist die Verbindung sehr unzuverlässig. Die Erfindung schafft ein chirurgisches Handgerät und einen Isoliereinsatz, der auf eine sichere sowie leicht lösbare Art und Weise mit einem Schaft des Handgeräts verbindbar ist. Das wird dadurch erreicht, dass der Isoliereinsatz (23) röhrchenartig ausgebildet ist und mit einem proximalen Endbereich an ein distales Ende eines rohrartigen Schaftes (13) des Handgeräts (10) lösbar gekoppelt ist. Dabei weist der proximale Endbereich des Isoliereinsatzes (23) mindestens zwei federnde Befestigungsmittel (26) auf, die jeweils ein zu einem Innenraum des Isoliereinsatzes (23) hinweisendes Rastelement (29) aufweisen.

## Beschreibung

Die Erfindung betrifft einen Isoliereinsatz für ein chirurgisches Handgerät gemäß dem Oberbegriff des Anspruchs 1. Des Weiteren betrifft die Erfindung ein chirurgisches Handgerät gemäß dem Anspruch 10 sowie ein Verfahren zur Handhabung eines chirurgischen Handgerätes gemäß dem Anspruch 15.

Chirurgische Handgeräte, insbesondere Resektoskope, der gattungsgemäßen Art werden vor allem in der Urologie bei elektrochirurgischen Arbeiten verwendet. Dabei kommen diese Geräte üblicherweise zur Resektion und Vaporisation von Gewebe, z. B. von Gewebe im unteren Harntrakt, zum Einsatz. Dazu kann das Handgerät, insbesondere das Resektoskop, ein längs verschiebbares elektrochirurgisches Durchgangsinstrument aufweisen, das nach dem Einführen des Geräts in den zu behandelnden Körper mit seinem distalen Arbeitsende aus einem distalen Ende eines Schaftrohres des Handgeräts hervorgeschoben werden kann. Das elektrochirurgische Durchgangsinstrument bzw. das Elektrodeninstrument weist dabei an einem distalen Ende eine elektrochirurgische Elektrode auf. Diese Elektrode kann beispielsweise die Form einer Schlinge oder eines Knopfes oder dergleichen umfassen.

Für die oben genannten Anwendungen wird die Elektrode mit einem hochfrequenten elektrischen Strom beaufschlagt. Dabei gilt es zu vermeiden, dass die Elektrode im elektrischen Kontakt mit dem Schaftrohr, insbesondere einem äußeren Schaftrohr bzw. einem Hüllrohr, des Handgerätes kommt. Bei einem derartigen elektrischen Kontakt könnte ein Kurzschluss einen Gerätedefekt verursachen oder zu unvorhersehbaren Schäden in dem zu behandelnden Körper führen. Um derartige Kurzschlüsse zu vermeiden, umfassen die Handgeräte bzw. Resektoskope an ihrem distalen Endbereich üblicherweise einen Abschnitt, der aus einem elektrisch isolierenden Material gefertigt ist. Dieser Abschnitt wird üblicherweise als Isolierspitze oder auch als Isoliereinsatz bezeichnet. Dabei kann der Isoliereinsatz entweder an einem inneren Schaft bzw. einem Schaftrohr, in dem ein Elektrodenträger geführt wird oder an dem äußeren Schaft bzw. dem Hüllrohr befestigt werden. Da diese Handgeräte bzw. die Resektoskope auf eine Mehrfachverwendung ausgelegt sind und dementsprechend regelmäßig zu sterilisieren bzw. zu autoklavieren sind, muss der Isoliereinsatz hohe Anforderungen an die Haltbarkeit und die Wiederaufbereitbarkeit erfüllen. Die Werkstoffauswahl ist daher in der Regel auf relativ teure Hochleistungskeramiken wie beispielsweise Siliziumnitrit beschränkt. Als Alternative zu diesen Keramiken werden elektrisch isolierende Kunststoffe eingesetzt. Diese Kunststoffe sind insbesondere thermostabil, damit sie bei einem Kontakt mit der Elektrode nicht sogleich schmelzen.

Als problematisch gestaltet sich die lösbare Kopplung derartiger Isoliereinsätze mit dem Schaft des Handgerätes. Bei lösbaren Verbindungen zwischen dem Isoliereinsatz und dem Schaft besteht grundsätzlich das Risiko, dass sich die Verbindung während der Behandlung unbeabsichtigt löst. Dies gilt es unter allen Umständen zu vermeiden, da dadurch nicht nur das Gerät an sich beschädigt werden kann, sondern auch die zu behandelnde Person verletzt werden könnte. Bei bekannten Befestigungsmechanismen von Isoliereinsätzen an beispielsweise einem inneren Schaft, muss stets ein Kompromiss hergestellt werden zwischen einer Haltekraft und einer Lösekraft. Zum einen muss die Befestigung des Isoliereinsatzes stark genug sein, um ein unbeabsichtigtes Lösen zu vermeiden. Zum anderen muss die bedienende Person den Isoliereinsatz noch mit einem vertretbaren Kraftaufwand vom Schaftende lösen können. Wird der Befestigungsmechanismus zu stark ausgebildet, erhöht sich zwar die Sicherheit gegenüber eines unbeabsichtigten Lösens, allerdings wird die Bedienperson Schwierigkeiten haben, den Isoliereinsatz für die oben genannten Zwecke zu lösen. Sitzt allerdings der Isoliereinsatz zu lose auf dem inneren Schaft, besteht, wie bereits ausgeführt, die Gefahr eines unbeabsichtigten Lösens.

Der Erfindung liegt daher die Aufgabe zugrunde, ein chirurgisches Handgerät, einen Isoliereinsatz sowie ein Verfahren zur Handhabung des chirurgischen Handgeräts zu schaffen, bei dem der Isoliereinsatz auf eine sichere sowie leicht lösbare Art und Weise mit einem Schaft des Handgeräts verbunden werden kann.

Eine Lösung dieser Aufgabe wird durch die Merkmale des Anspruchs 1 beschrieben. Demnach ist es vorgesehen, dass ein Isoliereinsatz für ein chirurgisches Handgerät, insbesondere für ein Resektoskop, röhrchenartig ausgebildet ist und mit einem proximalen Endbereich an ein distales Ende eines rohrartigen Schaftes des Handgeräts lösbar gekoppelt ist. Dabei weist der proximale Endbereich des Isoliereinsatzes mindestens zwei federnde Befestigungsmittel auf, die jeweils ein zu einem Innenraum des Isoliereinsatzes hinweisendes Rastelement aufweisen. Durch diese mindestens zwei Befestigungsmittel kann gewährleistet werden, dass der Isoliereinsatz ausreichend fest mit dem Schaft des Handgeräts koppelbar ist. Gleichermaßen lassen sich diese Befestigungsmittel durch die Bedienperson, ggf. mit einem Hilfswerkzeug, auf eine einfache und schnelle Art und Weise entkoppeln. Durch diese federnden Befestigungsmittel ist sowohl ein Befestigen des Isoliereinsatzes als auch ein Lösen des Isoliereinsatzes von dem chirurgischen Handgerät möglich.

Bevorzugt sieht es die Erfindung vor, dass zwei, vier, sechs oder mehr Befestigungsmittel an einem Umfang des proximalen Endbereichs gegenüberliegend angeordnet sind. Alternativ ist es darüber hinaus denkbar, dass drei oder mehr Befestigungsmittel an einem Umfang des proximalen Endbereichs des Isoliereinsatzes in einem Winkel von 120° bzw. in einem gleichen Winkel zueinander angeordnet sind. Insbesondere bei einer nicht konzentrischen bzw. axialen Form des Isoliereinsatzes bzw. der Positionierung auf dem inneren Schaft kann durch diese relative Positionierung der Befestigungsmittel an dem Umfang des Isoliereinsatzes eine verlässliche mechanische Verbindung hergestellt werden. Selbst bei einem leichten Verkippen des Isoliereinsatzes relativ zu dem Schaft kann durch die Positionierung der Befestigungsmittel ein ungewolltes Lösen verhindert werden. Durch diese Anordnung der Befestigungsmittel kann somit ein hohes Maß an Sicherheit und gleichzeitig eine einfache Handhabung gewährleistet werden.

Ein weiteres bevorzugtes Ausführungsbeispiel der Erfindung kann es vorsehen, dass die Befestigungsmittel an dem Umfang des Isoliereinsatzes als Schnapphaken ausgebildet sind. Diese Schnapphaken können laschenartige Federelemente aufweisen, an deren freien Enden Rastelemente angeordnet sind. Diese laschenartigen Federelemente sind aus der Wandung des proximalen Endbereichs des Einsatzes herausgebildet. Durch ihre laschenartige bzw. längliche Ausbildung weisen die Federelemente einen freien Federweg in radiale Richtung bezüglich des Schaftes bzw. des Einsatzes auf. So können für eine Verbindung des Einsatzes mit dem Schaft die Federelemente zunächst nach außen vorgespannt werden, bis die Rastelemente in komplementäre Öffnungen in dem Schaft arretiert sind. Die Federspannung der Federelemente bewirkt, dass diese ohne äußeren Krafteinfluss nicht aus der rastenden Haltestellung herausbewegt werden können. Zum Entkoppeln des Isoliereinsatzes von dem Schaft ist eine Kraft aufzuwenden, die gegen die federnde Rückstellkraft der Federelemente gerichtet ist. Da diese Kraft aufgrund der Dimensionierung des Materials verhältnismäßig gering ist, lässt sie sich von der Bedienperson problemlos aufbringen.

Vorzugsweise weist das Federelement gegenüber der Wandung des Isoliereinsatzes eine reduzierte Stärke auf. Gleichermaßen ist es denkbar, dass die Wandung des Isoliereinsatzes im proximalen Endbereich bzw. im Bereich der Befestigungsmittel bzw. zwischen den Befestigungsmitteln gegenüber der Wandung des Isoliereinsatzes eine reduzierte Stärke aufweist, vorzugsweise dass die Wandung des Isoliereinsatzes im proximalen Endbereich die gleiche Stärke aufweist, wie die Befestigungsmittel, insbesondere die Federelemente. Dadurch entsteht an der Innenwandung des Isoliereinsatzes ein ringartiger Absatz. Dieser Absatz teilt den Isoliereinsatz in einen Bereich mit einer reduzierten Wandstärke, in dem die Befestigungsmittel angeordnet sind und in einen Bereich mit einer größeren Wandstärke. Es ist denkbar, dass der Isoliereinsatz derart auf den inneren Schaft geschoben wird, dass dieser ringartige Absatz in Kontakt mit der rohrartigen Öffnung des inneren Schaftes tritt. Dadurch wird eine optimale relative Ausrichtung des Isoliereinsatzes und des Schaftes sichergestellt. Ein Verrutschen bzw. ein relatives Verkippen oder Wackeln kann dadurch vermieden werden. Bevorzugt ist die Stärke des distalen Bereiches des Isoliereinsatzes derart bemessen, dass der Übergang der Innenwandung des inneren Schaftes zu der Innenwandung des Isoliereinsatzes stufenlos, d. h. in einer Ebene, erfolgt.

Ein weiteres bevorzugtes Ausführungsbeispiel der vorliegenden Erfindung kann es vorsehen, dass die Befestigungsmittel paarweise nebeneinander und die Paare selbst gegenüberliegend an einem Umfang des proximalen Endbereichs angeordnet sind. Durch diese paarweise Anordnung der Befestigungsmittel kann die Sicherheit der lösbaren Verbindung weiter verbessert werden.

Insbesondere sieht es ein weiteres Ausführungsbeispiel vor, dass die, insbesondere keilförmigen, Rastelemente eine Höhe aufweisen, die mit der Wandstärke der Wandung des Isoliereinsatzes übereinstimmen. Darüber hinaus ist die Höhe der Rastelemente derart bemessen, dass sie bei der lösbaren Verbindung mit dem inneren Schaft nicht in den Innenraum des inneren Schaftes hineinragen. Das Profil bzw. ein Querschnitt der Rastelemente kann einen spitzen oder abgeflachten bzw. abgerundeten Keil darstellen.

Ein abgerundeter bzw. abgeflachter Keil kann für ein manuelles Abziehen des Isoliereinsatzes von dem inneren Schaft zu Reinigungszwecken und dergleichen vorteilhaft sein. Gleichermaßen bietet ein Rastelement mit einem dreieckigen Querschnitt oder sägezahnartigen Querschnitt eine größere Sicherheit bzgl. eines ungewollten Lösens. Die Kräfte zum Koppel und Halten der Federelemente sind auch abhängig von der Form und der Dimensionierung der Rastelemente. Durch Anpassung der Winkel der Rastelemente können die Kräfte zum Koppel und Halten der Federelemente unabhängig voneinander eingestellt werden.

Bevorzugt ist der hier beschriebene Isoliereinsatz aus einem Kunststoff hergestellt. Dieser Kunststoff ist sowohl elektrisch isolierend als auch thermostabil. Gleichermaßen ist es denkbar, dass der Isoliereinsatz aus einem beschichteten Metall hergestellt ist. Dabei bietet die Beschichtung des metallischen Isoliereinsatzes eine ausreichend große elektrische Isolation. Eine metallische Ausgestaltung des Isoliereinsatzes kann besonders vorteilhaft für die Langlebigkeit des Einsatzes sein.

Ein chirurgisches Handgerät zur Lösung der genannten Aufgabe weist die Merkmale des Anspruchs 10 auf. Dabei kann es sich bei dem chirurgischen Handgerät insbesondere um ein Resektoskop oder dergleichen handeln. Das beanspruchte chirurgische Handgerät weist einen inneren Schaft bzw. ein Schaftrohr sowie einen äußeren Schaft bzw. ein Hüllrohr auf. Das Hüllrohr ist um den inneren Schaft angeordnet. Einem distalen Ende des inneren Schaftes ist erfindungsgemäß ein Isoliereinsatz gemäß dem Anspruch 1 lösbar zugeordnet. Durch diese Anordnung des Isoliereinsatzes am distalen Ende sowohl des inneren Schaftes als auch des äußeren Schaftes kann verhindert werden, dass ein elektrochirurgisches Werkzeug bzw. eine Elektrode, die durch den inneren Schaft aus dem Isoliereinsatz herausgeführt wird, nicht in elektrischen Kontakt mit den metallisch ausgebildeten Schaftrohren tritt. Da der Isoliereinsatz aus einem elektrisch isolierenden Material hergestellt ist, ist eine Kontaktierung des elektrochirurgischen Werkzeuges mit dem Isoliereinsatz unkritisch.

Vorzugsweise ist es vorgesehen, dass ein äußerer Umfang des proximalen Endbereichs des Isoliereinsatzes derart dimensioniert ist, dass er über ein distales Ende des inneren Schaftes schiebbar ist. Dabei kann es vorgesehen sein, dass die Wandung des Isoliereinsatzes unterschiedliche Stärken aufweist, sodass die Innenwandung des Einsatzes einen ringartig umlaufenden Absatz aufweist, welcher beim Schieben auf den inneren Schaft in Kontakt mit dem ringartigen Ende des Schaftes tritt. Dadurch kann eine optimale Ausrichtung des Isoliereinsatzes an dem Schaft erreicht werden. Zusätzlich weist die Wandung des inneren Schaftes Öffnungen auf, in welche die Rastelemente vom Befestigungsmittel des Isoliereinsatzes für eine lösbare Kopplung eingreifen. Diese Öffnungen können umlaufend bzw. gegenüberliegend in der Wandung des Schaftes angeordnet sein. Dabei können diese Öffnungen die Form von Kreisen, Rechtecken bzw. Polyedern oder Ovale aufweisen. Die Öffnungen in der Wandung des Schaftes sind derart ausgebildet, dass sie mit der Form bzw. einem Querschnitt der Rastelemente korrespondieren. Die Öffnungen in der Wandung können Löcher in der Wandung darstellen oder lediglich trogartige Ausnehmungen in der Wandung, in welche die Rastelemente hineinschnappen können.

Weiter ist es denkbar, dass ein Abstand zwischen einer Innenseite des äußeren Schafts und der Außenseite des Isoliereinsatzes geringer ist als die Höhe der Rastelemente der Befestigungsmittel des Isoliereinsatzes. Dadurch, dass ein ringartiger Spalt wenigstens im Mittel zwischen dem Isoliereinsatz und dem äußeren Schaft eine geringere Breite aufweist als die Höhe der Rastelemente, wird der an den inneren Schaft gekoppelte Isoliereinsatz durch den äußeren Schaft gesichert. Durch diese Dimensionierung des äußeren Durchmessers des Isoliereinsatzes bzw. des Durchmessers des äußeren Schaftes, ist ein versehentliches Lösen der Befestigungsmittel aus den Öffnungen des inneren Schaftes nicht möglich. Selbst wenn die Befestigungsmittel bzw. die Rastelemente durch eine Krafteinwirkung bewegt werden würden, so würde das Befestigungsmittel bzw. die Federelemente an die Innenwandung des äußeren Schaftrohres drücken und zwar ohne dass sich dabei die Rastelemente aus den Öffnungen des inneren Schaftes lösen. Ein Lösen des Isoliereinsatzes von dem inneren Schaft ist somit nur möglich, wenn der innere Schaft mit dem Isoliereinsatz aus dem äußeren Schaft herausgezogen ist. Da die Schäfte jedoch während der Behandlung stets ineinander positioniert sind, kann es während der Behandlung bzw. während der Operation nicht zu einem unbeabsichtigten Lösen des Isoliereinsatzes von dem inneren Schaft kommen.

Weiter ist es denkbar, dass durch den inneren Schaft und/oder den Isoliereinsatz weitere Komponenten führbar bzw. leitbar sind, wie beispielsweise eine Optik, ein Elektrodeninstrument oder andere Werkzeuge. Dabei ist es denkbar, dass insbesondere ein Elektrodenträger auch durch entsprechende Führungen bzw. Aufnahmen in eine Wandung des Isoliereinsatzes geführt werden.

Ein Verfahren zur Handhabung eines chirurgischen Handgeräts zur Lösung der genannten Aufgabe weist die Maßnahmen des Anspruchs 16 auf. Demnach ist es vorgesehen, dass für die Verbindung des Isoliereinsatzes gemäß dem Anspruch 1 in einem Handgerät gemäß dem Anspruch 10 bzw. für die Vorbereitung zur Benutzung des Handgeräts zunächst der Isoliereinsatz mit einem proximalen Ende an ein distales Ende des inneren Schaftes gekoppelt und dann der innere Schaft mit dem Isoliereinsatz in den äußeren Schaft geführt wird und nach der Benutzung des Handgeräts der innere Schaft mit dem Isoliereinsatz aus dem äußeren Schaft herausgeführt wird und erst danach der Isoliereinsatz von dem inneren Schaft entkoppelt wird. Nur durch diese Reihenfolge der Installation bzw. des Zusammenbaus des Handgerätes kann ein ungewolltes Lösen des Isoliereinsatzes von dem inneren Schaft vermieden werden. Dabei sichert der äußere Schaft den Isoliereinsatz im zusammengebauten Zustand gegen ein Lösen der Befestigungsmittel. Sobald der äußere Schaft von dem inneren Schaft wegbewegt ist, kann der Isoliereinsatz auf die bekannte einfache Art und Weise von dem inneren Schaft abgekoppelt werden.

Ein bevorzugtes Ausführungsbeispiel der Erfindung wird nachfolgend anhand der Zeichnung näher beschrieben. In dieser zeigen:
- Fig. 1: eine schematische Darstellung eines chirurgischen Handgeräts, insbesondere eines Resektoskops,
- Fig. 2: eine schematische Darstellung eines distalen Endbereichs des chirurgischen Handgeräts,
- Fig. 3: eine perspektivische Darstellung eines Isoliereinsatzes,
- Fig. 4: eine perspektivische Darstellung des Isoliereinsatzes mit einem inneren Schaft,
- Fig. 5: eine Schnittdarstellung des Isoliereinsatzes mit dem inneren Schaft,
- Fig. 6: eine Darstellung des Isoliereinsatzes, und
- Fig. 7: eine Darstellung eines weiteren Ausführungsbeispiels eines Isoliereinsatzes.

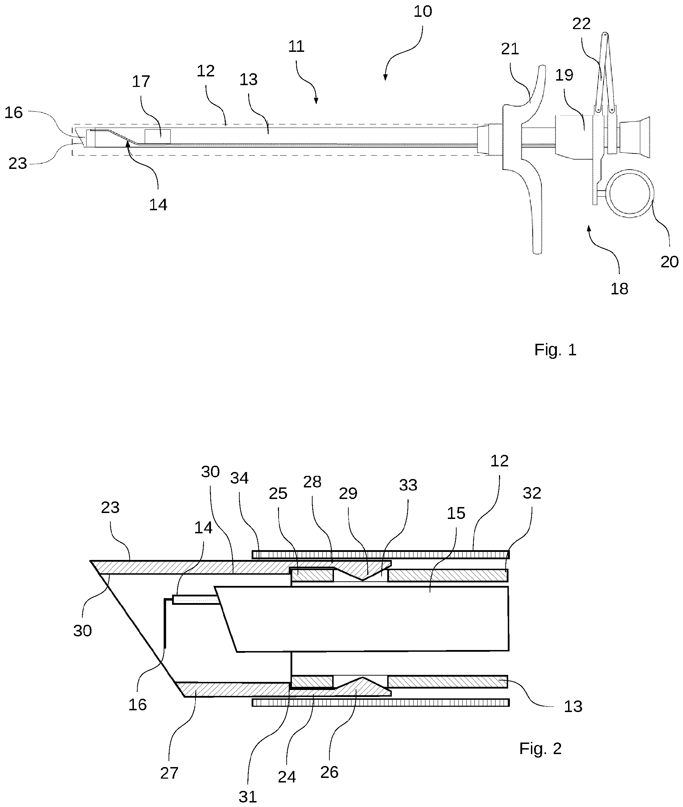

Fig. 1 zeigt eine schematische, seitliche Schnittdarstellung eines Resektoskops 10. Es soll ausdrücklich darauf hingewiesen werden, dass dieses Resektoskop 10 nur als ein mögliches Ausführungsbeispiel des beanspruchten chirurgischen Handgeräts dargestellt wird. Gleichermaßen ist es denkbar, dass es sich bei dem chirurgischen Handgerät um ein anderes Instrument als das hier dargestellte Resektoskop 10 handelt.

Das Resektoskop 10 weist einen Resektoskopschaft 11 auf, der einen dargestellten äußeren Schaft 12 bzw. Hüllrohr umfasst. Innerhalb des äußeren Schaftes 12 verläuft ein rohrartiger innerer Schaft 13. Innerhalb des inneren Schaftes 13 sind ein Elektrodeninstrument 14 sowie eine angedeutete Optik 15 dargestellt. Darüber hinaus können weitere hier nicht dargestellte Elemente in dem Resektoskop 10 verlaufen, wie beispielsweise ein separates Spülrohr und dergleichen.

Das Elektrodeninstrument 14 weist an einem distalen Ende ein elektrochirurgisches Werkzeug bzw. eine Elektrode 16 auf. Die hier dargestellte Elektrode ist als Schlinge dargestellt, kann jedoch auch als Knopf oder dergleichen ausgebildet sein. In der Fig. 1 ist erkennbar, dass das Elektrodeninstrument 14 durch ein Halteelement 17 mit einem teilkreisförmigen Querschnitt gegen Verschiebungen, d. h. Verschiebungen abweichend von der Längsrichtung des Resektoskopschaftes 11, geschützt ist. Das Elektrodeninstrument 14 ist längsverschiebbar in den inneren Schaft 13 gelagert. Das Haltelement 17 ist formkomplementär zur Innenwandung des inneren Schaftes 13 oder zur Außenwand der Optik 15 ausgebildet und weist eine teilzylindrische Form auf.

Das Elektrodeninstrument 14 kann durch Betätigung eines Handgriffs 18 zwangsgeführt axial in distale und proximale Richtung bewegt werden. Dabei kann es über das distale Ende des inneren Schaftes 13 und des äußeren Schaftes 12 hinausgeschoben werden. So wird es dem Operateur ermöglicht, auch weiter von der Resektoskopspitze entferntes Gewebe zu manipulieren. Zu diesem Zweck können ferner der innere Schaft 13 und/oder das Elektrodeninstrument 14 um ihre Längsachse drehbar gelagert sein. Für die Manipulation des Gewebes wird die Elektrode 16 mit einem hochfrequenten elektrischen Strom beaufschlagt.

Das in der Fig. 1 dargestellte Resektoskop 10 weist einen passiven Transporteur auf, bei dem ein Schlitten 19 durch Relativbewegung der proximal an dem Resektoskopschaft 11 angeordneten Griffteilen 20 und 21 gegen eine von einer Federbrücke 22 aufgebrachte Federkraft in distale Richtung gegen das distale, erste Griffteil 21 verschoben wird. Bei der Verschiebung des Schlittens 19 in distale Richtung gegen das Griffteil 21 wird das Elektrodeninstrument 14 in nicht dargestellter Weise in distale Richtung verschoben. Bei einer Entlastung der Handgriffteile 20, 21 zwingt die von der Federbrücke 22 erzeugte Federkraft den Schlitten 19 zurück in seine Ausgangsstellung, wobei das Elektrodeninstrument 14 in proximale Richtung gezogen wird. Bei der Rückverschiebung des Schlittens 19 kann ohne Handkraft des Operateurs, also passiv, ein elektrochirurgischer Eingriff mit der Elektrode 16 vorgenommen werden.

Bei bekannten chirurgischen Handgeräten ist der Isoliereinsatz an dem Schaft fest verbunden. Da dies jedoch ungünstig ist für eine Wiederverwendung bzw. für das Sterilisieren bzw. Reinigen existieren auch Ausführungsformen, bei denen die Isoliereinsätze lösbar an dem distalen Ende eines Schaftes verbunden sind. Bei den bekannten lösbaren Isoliereinsätzen besteht jedoch die Gefahr, dass sich diese während der Operation lösen. Andererseits, wenn die Verbindung des Einsatzes im Schaft stärker ausgebildet ist, erweist sich das Lösen des Einsatzes für eine Bedienperson als zu aufwendig.

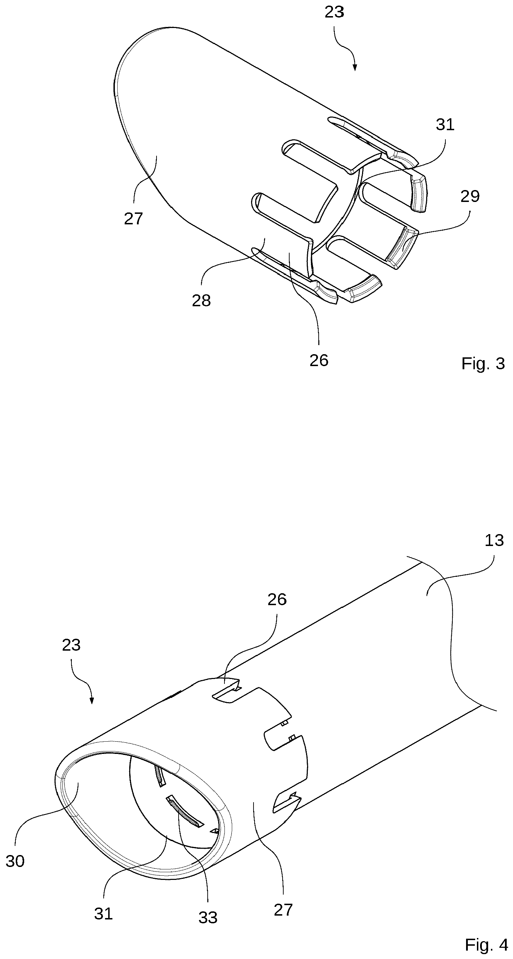

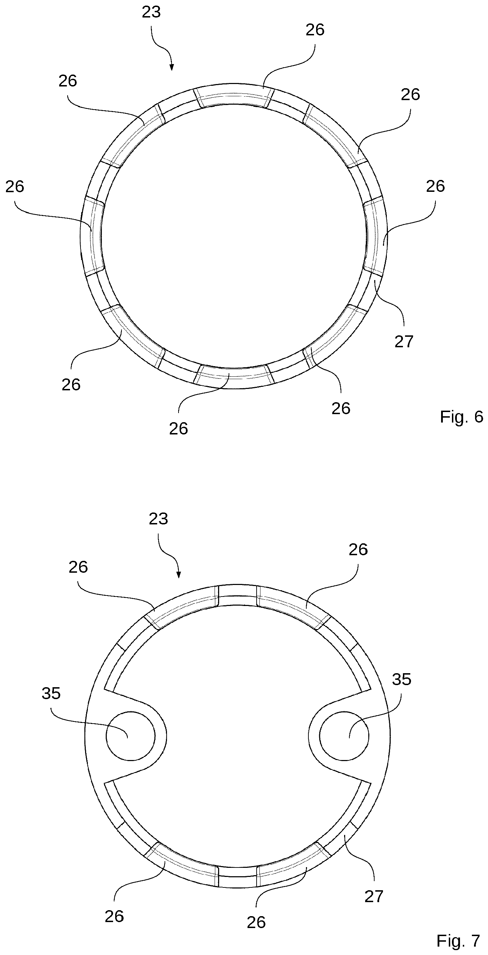

Der hier beschriebene erfindungsgemäße Isoliereinsatz 23 wird lösbar mit seinem proximalen Ende 24 an das distale Ende 25 des inneren Schaftes 13 gekoppelt (Fig. 2, hier wird nur ein distaler Bereich des Gerätes dargestellt). Dazu ist es vorgesehen, dass der Isoliereinsatz 23 an seinem proximalen Ende 25 Befestigungsmittel 26 aufweist (Fig. 3, 4). Diese Befestigungsmittel sind als Schnapphaken ausgebildet und aus der Wandung 27 des Isoliereinsatzes 23 ausgebildet. Dabei befinden sich die Befestigungsmittel 26, vorzugsweise paarweise, an gegenüberliegenden Positionen an dem Isoliereinsatz 23 (Fig. 7). Es ist außerdem denkbar, dass mehrere Befestigungsmittel 26 mit einem gleichen Winkelabstand zueinander der Wandung 27 des Isoliereinsatzes 23 zugeordnet sind (Fig. 6). Bei dem in der Fig. 7 dargestellten Ausführungsbeispiel eines Isoliereinsatzes 23 sind jeweils zwei Befestigungsmittel 26 paarweise an gegenüberliegenden Seiten der Wandung 27 des Einsatzes 23 angeordnet. Bei dem Ausführungsbeispiel eines Isoliereinsatzes 23 gemäß den Fig. 3 bis 6 befinden sich mehrere Befestigungsmittel 26 gegenüberliegend an der Wandung 27. Dabei weisen die Befestigungsmittel 26 untereinander jeweils einen gleichen Winkelabstand auf. Dadurch lässt sich eine besonders feste sowie positionsgenaue Verbindung herstellen.

Bei dem in den Fig. 7 dargestellten Ausführungsbeispiel für einen Isoliereinsatz 23 weist die Wandung 27 Aufweitungen auf, in denen Aufnahmen 35 für ein Elektrodeninstrument 14 angeordnet sind. Die hier dargestellten Aufnahmen 35 sind als lochartige bzw. bohrlochartige Durchgänge ausgebildet, durch welche beim Zusammenfügen der Komponenten des Geräts 10 das Elektrodeninstrument 14 führbar ist.

Die als Schnapphaken ausgebildeten Befestigungsmittel 26 bestehen im Wesentlichen aus einem stegartigen Federelement 28 und einem an dem freien Ende des Federelements 28 angeordneten Rastelement 29. Dieses Rastelement 29 kann, wie in dem Querschnitt der Fig. 2 dargestellt, keilförmig ausgebildet sein. Es ist jedoch auch denkbar, dass das Rastelement 29 abgerundet ausgebildet ist.

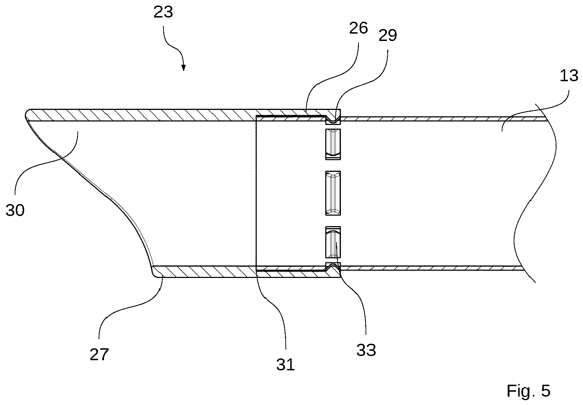

Die Bereiche der Wandung 27, die an dem proximalen Ende 24 des Isoliereinsatzes 23 zwischen dem Befestigungsmittel 26 bzw. den Federelementen 28 angeordnet sind, sind zum einen von dem Befestigungsmittel 26 getrennt, damit diese frei federn können und zum anderen weisen diese Bereiche die gleiche Stärke auf wie die Federelemente 28. Dadurch entsteht an einer Innenseite 30 des Isoliereinsatzes 23 ein Absatz 31. Dieser Absatz 31 erstreckt sich ringartig um die gesamte Innenseite 30 des Isoliereinsatzes 23. Bei der Verbindung des Isoliereinsatzes 23 mit dem inneren Schaft 13 wird der Isoliereinsatz 23 soweit auf den Schaft 13 geschoben, bis der Absatz 31 gegen das distale Ende 25 des inneren Schaftes 13 stößt (Fig. 4 und 5).

Der innere Schaft 13 weist in seiner Wandung 32 am distalen Ende 25 Öffnungen 33 auf (Fig. 4 und 5). Diese Öffnungen 33 sind derart dimensioniert sowie positioniert, dass die Befestigungsmittel 26 bzw. die Rastelemente 29 des Isoliereinsatzes 23 beim Aufeinanderschieben des Schaftes 13 und des Isoliereinsatzes 23 zusammenschnappen. Bei diesem Übereinanderschieben des Isoliereinsatzes 23 und des inneren Schaftes 13 werden die Federelemente 28 durch die Rastelemente 29 zunächst nach außen hin bewegt und aufgeweitet, bis die Rastelemente 29 in die Öffnungen 33 schnappen. In Zusammenwirkung mit dem Absatz 31 befindet sich der Isoliereinsatz 23 in der optimalen Position an dem distalen Ende 25 des inneren Schaftes 13. Für die Demontage muss eine Bedienperson nun die Rastelemente 29 aus den Öffnungen 33 wegbewegen bzw. die Federelemente 28 aufweiten und den Isoliereinsatz 23 von dem inneren Schaft 13 abziehen.

Damit jedoch insbesondere während einer Operation, der Isoliereinsatz 23 nicht ungewollt von dem inneren Schaft 13 gelöst wird, wird nach dem Zusammenfügen über den inneren Schaft 13 und teilweise auch über den Isoliereinsatz 23 der äußeren Schaft 12 geschoben (Fig. 2). Erfindungsgemäß ist der Innendurchmesser des äußeren Schaftes 12 bzw. der Außendurchmesser des Isoliereinsatzes 23 derart beschaffen, dass ein Spalt 34 bzw. ein Ringraum zwischen einer Innenwandung des Außenschaftes 12 und der Wandung 27 des Isoliereinsatzes 23 kleiner ist als die Höhe der Rastelemente 29. Durch diesen engen Spalte 34 bzw. diesen geringen Spielraum werden die Rastelemente 29 in den Öffnungen 33 fixiert. Selbst wenn auf den Isoliereinsatz 23 eine mechanische Kraft ausgeübt wird, können die Befestigungsmittel 26 bzw. die Federelemente 28 nicht derart aufgeweitet werden, dass sich der Isoliereinsatz 23 von dem inneren Schaft 13 löst. Diese umhüllende Wirkung des Außenschaftes 12 um den Isoliereinsatz 23 bewirkt, dass der Einsatz 23 in seiner relativen Ausrichtung zu dem inneren Schaft 13 fixiert bleibt. Erst wenn der Außenschaft 12 wieder von dem inneren Schaft 13 abgezogen wurde, kann die Bedienperson den Isoliereinsatz 23 von dem inneren Schaft 13 lösen.

Ein bevorzugtes Ausführungsbeispielsweise kann es vorsehen, dass der Isoliereinsatz 23 mehrere Befestigungselemente 26 aufweist und der innere Schaft 13 entsprechend mehrere Öffnungen 33. Neben der hier dargestellten Form des Isoliereinsatzes 23 sind auch noch weitere Formen, wie beispielsweise ein gerades distales Ende, denkbar.

**Bezugszeichenliste**

| | | | |
|---|---|---|---|
| 10 | Resektoskop | 29 | Rastelement |
| 11 | Resektoskopschaft | 30 | Innenseite |
| 12 | äußerer Schaft | 31 | Absatz |
| 13 | innerer Schaft | 32 | Wandung |
| 14 | Elektrodeninstrument | 33 | Öffnung |
| 15 | Optik | 34 | Spalt |
| 16 | Elektrode | 35 | Aufnahme |
| 17 | Haltelement | | |
| 18 | Handgriff | | |
| 19 | Schlitten | | |
| 20 | Griffteil | | |
| 21 | Griffteil | | |
| 22 | Federbrücke | | |
| 23 | Isoliereinsatz | | |
| 24 | proximales Ende | | |
| 25 | distales Ende | | |
| 26 | Befestigungsmittel | | |
| 27 | Wandung | | |
| 28 | Federelement | | |

## Patentansprüche

1. Isoliereinsatz (23) für ein chirurgisches Handgerät, insbesondere für ein Resektoskop (10), wobei der röhrchenartige Isoliereinsatz (23) mit einem proximalen Endbereich (24) an ein distales Ende (25) eines rohrartigen Schaftes (13) des Handgeräts lösbar koppelbar ist, **dadurch gekennzeichnet, dass** der proximale Endbereich (24) des Isoliereinsatzes (23) mindestens zwei federnde Befestigungsmittel (26) aufweist, die jeweils ein zu einem Innenraum des Isoliereinsatzes (23) hinweisendes Rastelement (29) aufweisen.

2. Isoliereinsatz (23) für ein chirurgisches Handgerät nach Anspruch 1, **dadurch gekennzeichnet, dass** zwei oder vier oder sechs oder mehr Befestigungsmittel (26) an einem Umfang des proximalen Endbereichs (24) gegenüberliegend angeordnet sind.

3. Isoliereinsatz (23) für ein chirurgisches Handgerät nach Anspruch 1, **dadurch gekennzeichnet, dass** drei oder mehr Befestigungsmittel (26) an einem Umfang des proximalen Endbereichs (24) in einem Winkel von 120° bzw. in gleichen Winkeln zueinander angeordnet sind.

4. Isoliereinsatz (23) für ein chirurgisches Handgerät nach einem der vorangegangenen Ansprüche, **dadurch gekennzeichnet, dass** die Befestigungsmittel (26) als Schnapphaken ausgebildet sind.

5. Isoliereinsatz (23) für ein chirurgisches Handgerät nach Anspruch 4, **dadurch gekennzeichnet, dass** der Schnapphaken ein laschenartiges Federelement (28) aufweist, das aus einer Wandung (27) des proximalen Endbereichs (24) des Einsatzes (23) herausgebildet ist, wobei an einem freien Ende des Federelementes (28) das Rastelement (29) angeordnet ist.

6. Isoliereinsatz (23) für ein chirurgisches Handgerät nach Anspruch 5, **dadurch gekennzeichnet, dass** das Federelement (28) gegenüber der Wandung (27) des Isoliereinsatzes (23) eine reduzierte Stärke aufweist.

7. Isoliereinsatz (23) für ein chirurgisches Handgerät nach einem der vorangegangenen Ansprüche, **dadurch gekennzeichnet, dass** die Wandung (27) des Isoliereinsatzes (23) im proximalen Endbereich (24) bzw. im Bereich der Befestigungsmittel (26) bzw. zwischen den Befestigungsmitteln (26) gegenüber der Wandung (27) des Isoliereinsatzes (23) eine reduzierte Stärke aufweist, vorzugsweise dass die Wandung (27) des Isoliereinsatzes (23) im proximalen Endbereich (24) die gleiche Stärke aufweist, wie die Befestigungsmittel (26), insbesondere die Federelemente (28).

8. Isoliereinsatz (23) für ein chirurgisches Handgerät nach einem der vorangegangenen Ansprüche, **dadurch gekennzeichnet, dass** die Befestigungsmittel (26) paarweise nebeneinander und die Paare gegenüberliegend an einem Umfang des proximalen Endbereichs (24) angeordnet sind.

9. Isoliereinsatz (23) für ein chirurgisches Handgerät nach einem der vorangegangenen Ansprüche, **dadurch gekennzeichnet, dass** die, insbesondere keilförmigen, Rastelemente (29) eine Höhe aufweisen, die mit der Wandstärke der Wandung (27) des Isoliereinsatzes (23) übereinstimmt.

10. Chirurgisches Handgerät, insbesondere Resektoskop (10), mit einem inneren Schaft (13) bzw. einem Schaftrohr und einem äußeren Schaft (12) bzw. einem Hüllrohr, und einem Isoliereinsatz (23) gemäß dem Anspruch 1, wobei der Isoliereinsatz (23) mit dem inneren Schaft (13) lösbar koppelbar ist und der äußere Schaft (12) über den inneren Schaft (13) und den Isoliereinsatz (23) bewegbar ist.

11. Chirurgisches Handgerät nach Anspruch 10, **dadurch gekennzeichnet, dass** ein äußerer Umfang eines proximalen Endbereichs (24) des Isoliereinsatzes (23) derart dimensioniert ist, dass er über ein distales Ende (25) des inneren Schafts (13) schiebbar ist.

12. Chirurgisches Handgerät nach Anspruch 10 oder 11, **dadurch gekennzeichnet, dass** eine Wandung (27) des inneren Schafts (13) Öffnungen (33) aufweist, in die Rastelemente (29) von Befestigungsmitteln (26) des Isoliereinsatzes (23) für eine lösbare Kopplung des Isoliereinsatzes (23) mit dem inneren Schaft (13) eingreifen.

13. Chirurgisches Handgerät nach einem der Ansprüche Anspruch 10 bis 12, **dadurch gekennzeichnet, dass** ein Abstand zwischen einer Innenseite des äußeren Schafts (12) und der Außenseite des Isoliereinsatzes (23) geringer ist als die Höhe von Rastelementen (29) der Befestigungsmittel (26) des Isoliereinsatzes (23).

14. Chirurgisches Handgerät nach einem der Ansprüche Anspruch 10 bis 12, **dadurch gekennzeichnet, dass** durch den inneren Schaft (13) und/oder den Isoliereinsatz (23) weitere Komponenten führbar bzw. leitbar sind, wie beispielsweise eine Optik (15), ein Elektrodeninstrument (14) oder andere Werkzeuge.

15. Verfahren zur Handhabung eines chirurgischen Handgerätes, insbesondere eines Resektoskopes (10), gemäß Anspruch 10 mit einem inneren Schaft (13) bzw. einem Schaftrohr und einem äußeren Schaft (12) bzw. einem Hüllrohr und einem Isoliereinsatz (23), insbesondere gemäß Anspruch 1, **dadurch gekennzeichnet, dass** für eine Vorbereitung zur Benutzung des Handgerätes zunächst der Isoliereinsatz (23) mit einem proximalen Ende (24) an ein distales Ende (25) des inneren Schaftes (13) gekoppelt und dann der innere Schaft (13) mit dem Isoliereinsatz (23) in den äußeren Schaft (12) geführt wird und nach der Benutzung des Handgerätes der innere Schaft (13) mit dem Isoliereinsatz (23) aus dem äußeren Schaft (12) geführt und dann der Isoliereinsatz (23) von dem inneren Schaft (13) entkoppelt wird.
